# EUROPEAN PATENT APPLICATION

(11) **EP 1 902 710 A1**
(43) Date of publication of application: **26.03.2008**
(21) Application number: 06767637.9
(22) Date of filing: 29.06.2006
(51) Int. Cl.: A61K 31/198, A23L 1/30, A23L 1/302, A23L 1/305, A61K 31/355, A61K 31/375, A61K 31/522, A61K 31/525, A61P 3/06, A61P 3/10, A61P 7/02, A61P 9/10, A61P 9/12, A61P 9/14, A61P 11/00, A61P 15/08, A61P 15/10, A61P 15/12, A61P 25/28, A61P 37/04

(54) **PHARMACEUTICAL COMPOSITION AND BEVERAGE COMPOSITION COMPRISING L-ARGININE**

(30) Priority: 04.07.2005 JP 2005195412
(71) Applicant: Tokyo Medical and Dental University, Tokyo 113-8510 (JP)
(72) Inventor: AZUMA, Hiroshi Tokyo Medical and Dental University, Tokyo 113-8510 (JP)
(74) Representative: Hirsch & Associés
(86) International application number: PCT/JP2006/313020
(87) International publication number: WO 2007/004523

(57) **Abstract**

A pharmaceutical composition and a beverage composition comprising the following three components (A), (B) and (C): (A) L-arginine or a salt thereof; (B) caffeine or a salt thereof; and (C) at least one substance selected from the group consisting of vitamin C or a derivative thereof, vitamin E or a derivative thereof and folic acid or a derivative thereof. The compositions show an effect of L-arginine even at a low L-arginine content, and therefore can be suitably used for the prevention, amelioration and/or treatment of at least one disease selected from the group consisting of arteriosclerosis, angiectasia, hyperlipemia, climacteric disorder, diabetes, angina pectoris, hypertension, erectile dysfunction, thrombosis, immunodeficiency, dementia, gestosis, respiratory failure and hemorrhoid, in the form of a pharmaceutical preparation or a beverage.

## Description

### TECHNICAL FIELD

The present invention relates to a pharmaceutical composition and a beverage composition including L-arginine.

### BACKGROUND ART

Male patients with penile erectile dysfunction are said to be increasing rapidly with the growth of elderly population. Erection occurs through relaxation of the corpus cavernosum tissue, increase in penile artery blood flow, and suppression of diapedesis from the penile vein. It was revealed that such relaxation of the corpus cavernosum, increase in penile artery blood flow, and the like may be caused by the following mechanisms. More specifically, nitrogen monoxide (NO) synthesized in endothelial cells by NO synthase is diffused into smooth muscle cells that exist in the penile corpus cavernosum. Next, NO binds to a heme component of guanylate cyclase to activate the enzyme, whereby cyclic guanosine monophosphate (cGMP) is synthesized from guanosine triphosphate. Since calcium content in smooth muscle cells is decreased by cGMP, relaxation of the smooth muscle proceeds with an increase in cGMP content, thereby resulting in an increase of penile artery blood flow.

When any one of the processes as described above is inhibited, erectile dysfunction can be developed. In addition, phosphodiesterase-5 (PDE-5) that is localized in the penile corpus cavernosum is a cGMP-degrading enzyme, and it is known that an increase in PDE-5 content may result in development of erectile dysfunction. In this respect, sildenafil citrate (hereinafter, abbreviated as "sildenafil") is a PDE-5 inhibitor approved by FDA, and has been manufactured as an orally administrable therapeutic agent for erectile dysfunction. However, the efficacy ratio of sildenafil is only 50%. Moreover, due to the absence of selectivity for the blood vessel, blood flow is increased thereby, and it also leads to problems of side effects.

Meanwhile, L-arginine is expected to have an amelioration effect of erectile dysfunction by an NO production stimulatory effect since it is a substrate of nitrogen monoxide synthase in endothelial cells.

The following Patent Document 1 discloses that use of L-arginine in combination with caffeine as an agent for ameliorating erectile dysfunction synergistically increases blood flow of the corpus cavernosum, and thus a pharmaceutical formulation including the same is useful as an ameliorating agent of erectile dysfunction.

Additionally, among causes of death segregated by age, cardiac diseases and cerebrovascular disorders rank at the top for the age bracket of at least 80 years olds. Ischemic cardiac diseases (e.g., myocardial infarction and angina pectoris) accounting for a large proportion of the cardiac diseases, and cerebral infarction accounting for about a half of the cerebral blood vessel diseases can be developed based on arteriosclerosis. Therefore, prevention and treatment of arteriosclerosis have been significant issues.

With regard to arteriosclerosis, LDL cholesterol first invades the inner wall of a damaged blood vessel, and the LDL cholesterol is converted into oxidized LDL by an action of active oxygen. Uptake of the oxidized LDL by macrophage yields foam cells. Then, hyperplasia and thickening of thus resulting cells cause arteriosclerosis.

NO produced from L-arginine in bodies is expected to exhibit an effect of preventing arteriosclerosis since it is a potent radical scavenger that assists prevention of LDL cholesterol from oxidization to be converted into oxidized LDL, and due to the actions of suppressing smooth muscle cell proliferation, suppressing platelet aggregation, lowering blood pressure, and the like.

The following Patent Document 2 discloses, as a pharmaceutical formulation effective for arteriosclerosis, a pharmaceutical formulation including L-arginine or a pharmaceutically acceptable salt thereof as a principal component.

Patent Document 1: Japanese Unexamined Patent Application, First Publication No. 2001-81032

Patent Document 2: Japanese Unexamined Patent Application, First Publication No. Hei 5-163139

### DISCLOSURE OF THE INVENTION

### Problems to be Solved by the Invention

However, the pharmaceutical formulations in the Patent Documents 1 and 2 require administration or intake of a large quantity of L-arginine for achieving its effect. Therefore, side effects have been problematic in these cases. Moreover, in order to take such a composition conveniently, application to beverages has been also expected.

The present invention was made in view of the foregoing problems, and an object of the invention is to provide a pharmaceutical composition and a beverage composition that show an effect of L-arginine even at a low L-arginine content without the accompanying a side effect.

### Means for Solving the Problems

The present inventors thoroughly investigated in order to solve the aforementioned problems, and consequently found that the problems can be solved by further including at least one substance selected from vitamin C or a derivative thereof, vitamin E or a derivative thereof, and folic acid or a derivative thereof in addition to L-arginine and caffeine, thereby enhancing the effect of L-arginine. Accordingly, the present invention was accomplished. More specifically, the present invention provides the following aspects.

In a first aspect, a pharmaceutical composition includes the following components (A), (B) and (C): (A) L-arginine or a salt thereof; (B) caffeine or a salt thereof; and (C) at least one substance selected from the group consisting of vitamin C or a derivative thereof, vitamin E or a derivative thereof and folic acid or a derivative thereof.

According to the pharmaceutical composition of the present invention, at least one substance selected from the group consisting of vitamin C or a derivative thereof, vitamin E or a derivative thereof, and folic acid or a derivative thereof is included. Use of this component (C) in combination can further enhance the effect of L-arginine, whereby a pharmaceutical composition can be provided which shows the effect at a low L-arginine content.

In a second aspect of the pharmaceutical composition according to the first aspect, at least two substances are used in combination as the component (C). In a third aspect of the pharmaceutical composition according to the second aspect, at least the vitamin E or a derivative thereof, and the folic acid or a derivative thereof are used in combination as the component (C).

According to these aspects, because at least two substances, particularly vitamin E or a derivative thereof and folic acid or a derivative thereof are used in combination as the component (C), the effect of L-arginine can be further enhanced.

In a fourth aspect of the present invention, the pharmaceutical composition according to any one of the first to third aspects is used for the prevention, amelioration and/or treatment of at least one disease selected from the group consisting of arteriosclerosis, angiectasia, hyperlipemia, climacteric disorder, diabetes, angina pectoris, hypertension, erectile dysfunction, thrombosis, immunodeficiency, dementia, gestosis, respiratory failure and hemorrhoid.

Since the pharmaceutical composition of the present invention exhibits the effect to enhance L-arginine actions, it is particularly efficacious as a pharmaceutical composition for use in the prevention, amelioration and/or treatment of at least one disease selected from the group described above for the following reasons.

Use of L-arginine in the prevention, amelioration and/or treatment of the aforementioned disease requires administration or intake of a large quantity of L-arginine. In such cases, therefore, side effects have been problematic. However, according to the pharmaceutical composition of the present invention, the effect of L-arginine actions can be synergistically potentiated. Therefore, the amount of NO can be increased at a low L-arginine content. NO biosynthesized using L-arginine as a substrate has a variety of physiological roles, and in addition to the angiectactic action, suppressive action of platelet aggregation and adhesion, suppressive action of LDL cholesterol oxidization, maintaining action of immunity, improving action of memory via increase in blood supply, regulation of insulin secretion, actions of bronchiolar smooth muscular dilatation and remission of anal sphincter muscular abnormal contraction as well as increasing action of circulating blood, etc. have been known so far (Reference Document: "The ARGININE SOLUTION" The First Guide to America's New Cardio-Enhancing Supplement: Robert Fried, PhD and Woodson C Merrell, MD with James Thornton, Warner Books, Inc., 1271 Avenue of the Americas, New York, NY 10020 (1999)). Therefore, the pharmaceutical composition of the present invention can be suitably used as a pharmaceutical composition for use in the prevention, amelioration and/or treatment of the aforementioned diseases at a low L-arginine content.

In a fifth aspect of the present invention, the pharmaceutical composition according to any one of the first to fourth aspects includes 0.05 to 67 moles of the component (B); and 0.05 to 67 moles of vitamin C or a derivative thereof, 0.0005 to 67 moles of vitamin E or a derivative thereof, and/or or 0.005 to 67 moles of folic acid or a derivative thereof as the component (C), per 100 moles of the component (A).

According to this aspect, the L-arginine actions can be enhanced by making the blend ratio fall within the above range, in which a pharmaceutical composition that shows the effect at a low L-arginine content can be provided.

In a sixth aspect of the present invention, a beverage composition includes the following components (A), (B) and (C): (A) L-arginine or a salt thereof; (B) caffeine or a salt thereof; and (C) at least one substance selected from the group consisting of vitamin C or a derivative thereof, vitamin E or a derivative thereof and folic acid or a derivative thereof.

According to a seventh aspect of the present invention, in the beverage composition according to the sixth aspect, at least two substances are used in combination as the component (C).

According to an eighth aspect of the present invention, in the beverage composition according to the seventh aspect, at least the vitamin E or a derivative thereof, and the folic acid or a derivative thereof are used in combination as the component (C).

According to a ninth aspect, the beverage composition according to any one of sixth to eighth aspects comprises 0.05 to 67 moles of the component (B); and 0.05 to 67 moles of vitamin C or a derivative thereof, 0.0005 to 67 moles of vitamin E or a derivative thereof, and/or 0.005 to 67 moles of folic acid or a derivative thereof as the component (C), per 100 moles of the component (A).

According to the beverage composition of the present invention, because the effect of L-arginine can be enhanced similarly to the pharmaceutical composition, a beverage composition and a beverage can be produced which show an effect of L-arginine even at a low L-arginine content. In addition, since it is a beverage, it can be readily taken. Such a beverage can be used as, for example, a health food.

### Effects of the Invention

According to the present invention, the effect of L-arginine is enhanced, whereby a pharmaceutical composition and a beverage composition used for the prevention, amelioration and/or treatment of at least one disease selected from the group consisting of arteriosclerosis, angiectasia, hyperlipemia, climacteric disorder, diabetes, angina pectoris, hypertension, erectile dysfunction, thrombosis, immunodeficiency, dementia, gestosis, respiratory failure and hemorrhoid even at a low L-arginine content can be provided.

### BRIEF DESCRIPTION OF THE DRAWINGS

Fig. 1 illustrates an administration schedule of the various drugs;
Fig. 2 shows a view illustrating a relationship of the vitamin C concentration and the relaxation rate in Examples 1 to 3;
Fig. 3 shows a relationship of the vitamin E concentration and the relaxation rate in Examples 4 to 6;
Fig. 4 shows a relationship of the folic acid concentration and the relaxation rate in Examples 7 to 9; and
Fig. 5 shows a relationship of the relaxation rate with the substances vitamin C, vitamin E, and folic acid added alone or as a mixture of two or more thereof in Examples 10 to 15.

### DESCRIPTION OF REFERENCES SIGNS

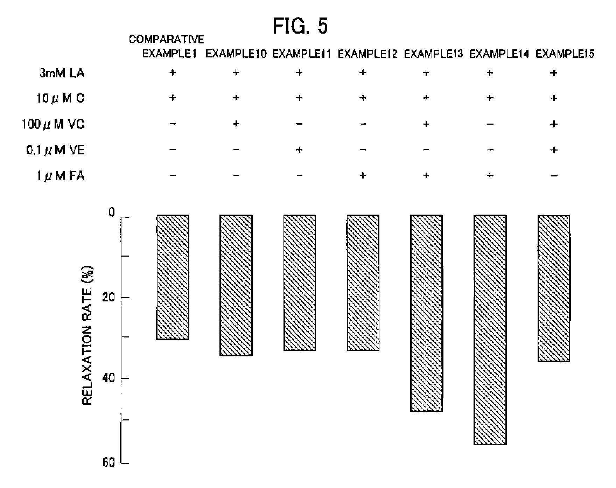

In Fig. 5, signs denote the following compounds, respectively:
LA: L-arginine,
C : caffeine,
VC: ascorbic acid,
VE: a-tocopherol, and
FA: folic acid.

### PREFERRED MODE FOR CARRYING OUT THE INVENTION

The pharmaceutical composition and the beverage composition of the present invention include the following components: (A) L-arginine or a salt thereof; (B) caffeine or a salt thereof; and (C) at least one substance selected from the group consisting of vitamin C or a derivative thereof, vitamin E or a derivative thereof, and folic acid or a derivative thereof. Hereinafter, each constitutive component is explained.

### Component (A): L-arginine or Salt thereof

Examples of the salt of L-arginine used in the present invention include mineral acid salts such as hydrochloride, sulfate and nitrate, and organic acid salts such as acetate and citrate.

### Component (B): Caffeine or Salt thereof

As the salt of caffeine for use in the present invention, a similar salt to that of L-arginine can be used.

### Component (C): Vitamin C or Derivative thereof

The vitamin C or a derivative thereof for use in the present invention is not particularly limited, and for example, ascorbyl palmitate, Mg-ascorbyl phosphate, ascorbyl acetate or the like can be used.

### Vitamin E or Derivative thereof

The vitamin E or a derivative thereof for use in the present invention is not particularly limited, and for example, α-tocopherol acetate or the like can be used.

### Folic acid or Derivative thereof

The folic acid or a derivative thereof for use in the present invention is not particularly limited, and commonly used folic acid or a derivative thereof can be used.

Additionally, in the pharmaceutical formulation or beverage for the prevention, amelioration and/or treatment of the present invention, sildenafil citrate may be used in combination, in addition to the components as described above. Blend Ratio

In the pharmaceutical composition and beverage composition of the present invention, with respect to the blend ratio (molar ratio) of the components (A): L-arginine or a salt thereof, (B): caffeine or a salt thereof, and (C): at least one substance selected from vitamin C or a derivative thereof, vitamin E or a derivative thereof, and folic acid or a derivative thereof in the composition, when the component (A): L-arginine or a salt thereof is estimated to account for 100, the component (B): caffeine or a salt thereof accounts for preferably from 0.05 to 67, and more preferably from 0.5 to 20, in light of improvement of the effect of L-arginine. Moreover, when the component (C) is the vitamin C or a derivative thereof, it accounts for preferably from 0.05 to 67, and more preferably from 0.5 to 20. When the component (C) is the vitamin E or a derivative thereof, it accounts for preferably from 0.0005 to 67, and more preferably from 0.001 to 20. Meanwhile, when the component (C) is the folic acid or a derivative thereof, it accounts for 0.005 to 67.

In addition, two or more substances can be used in combination as the component (C), and the most preferable combination when two or more substances are used is a combination of the vitamin E or a derivative thereof, and the folic acid or a derivative thereof.

### Pharmaceutical Formulation

### Dosage Form

The pharmaceutical formulation of the present invention can be produced into a variety of administration forms through blending necessary additives, in addition to the pharmaceutical composition. When a solid oral formulation is prepared, after adding an excipient and a binding agent, as well as a disintegrant, a lubricant, a colorant, a seasoning agent, a flavoring agent and the like as needed, tablets, granules, powdered formulations, capsules and the like can be produced by a conventional method. When an oral liquid formulation is prepared, a seasoning agent, a buffer agent, a stabilizing agent, a flavoring agent and the like may be addedto enable production of internal liquid formulations, syrup agents, jelly agents, elixirs and the like by a conventional method. When an injectable formulation is prepared, a pH-adjusting agent, a buffer agent, a stabilizing agent, an isotonizing agent, a topical anesthetic agent and the like may be added to enable production of injectable formulations for subcutaneous, intramuscular, intravenous and penile intracavernousal use and the like by a conventional method. When an ointment is prepared, a base, a stabilizer, a wetting agent, a preservative and the like commonly used may be blended as needed, and can be mixed to be formulated by a conventional method. When a patch is prepared, the ointment, cream, gel, paste or the like may be applied to a common support by a conventional method. Furthermore, the pharmaceutical formulation of the present invention can be in the form of a kit produced by separately preparing the component (A), the component (B), and the component (C). Effective Dose

The effective dose of the pharmaceutical formulation varies depending on the patient's body weight, age, sex, administration method, body conditions, symptoms, dosage forms, and the like. When it is orally administered to an adult, the total dose of the components (A), (B) and (C) is preferably from 0.2 g to 5 g per day, which may be received once, or in two to several divided dosage forms. In the case of the patch, a tape or the like including from 0.02 g to 1 g for one is preferably used. Moreover, in the case of a liniment, an ointment cream or the like containing from 0.02 g to 1 g for one may be used.

The vitamin C or a derivative thereof, vitamin E or a derivative thereof, and folic acid or a derivative thereof included in the pharmaceutical composition show an effect to enhance the effect of L-arginine; therefore, the pharmaceutical formulation of the present invention is a useful formulation for the prevention, amelioration and/or treatment of at least one disease selected from the group consisting of arteriosclerosis, angiectasia, hyperlipemia, climacteric disorder, diabetes, angina pectoris, hypertension, erectile dysfunction, thrombosis, immunodeficiency, dementia, gestosis, respiratory failure, and hemorrhoid.

### Beverage

### Form

The beverage according to the present invention can be used through blending an excipient, an expander, a binding agent, a thickening agent, an emulsifying agent, a colorant, a flavor, a food additive, a seasoning and the like, in addition to the beverage composition. The beverage form is not particularly limited, and can be formed into paste or liquid states, capsules such as hard capsules and soft capsules, tablets, pills, as well as powdery, granular, and lozenge shapes. These can be produced in accordance with a general production method.

Furthermore, these may be taken either directly, or after dissolving in water, hot water, milk or the like to achieve their shape or preference.

### Effective Intake

Moreover, the effective intake of the beverage may vary depending on the age, sex, taking method, form and the like, and is preferably from 0.02 g to 10 g and more preferably from 0.2 g to 5 g for one in terms of the total amount of the components (A), (B) and (C).

### EXAMPLES

Hereinafter, the present invention is explained in more detail by way of Examples, but the present invention is not to be limited to the following Examples.

### Examination of Effects of Added Vitamin C (VC, ascorbic acid) on Rabbit Penile Corpus cavernosum Dilator Response

### Examples 1 to 3, Comparative Example 1

A male Japanese native white rabbit weighing about 2.5 kg was killed by bleeding from the femoral artery under anesthesia with sodium pentobarbital (25 mg/kg, intravenous injection), and thereafter the penis was extirpated. The tunica albuginea was removed in an ice-cooled Krebs solution, and the penile corpus cavernosum was separated to prepare a columnar specimen having a length of about 7 mm, and a diameter of about 2 mm. One end of the specimen was fixed, and the other end was connected to a force displacement transducer for determining tensile force (manufactured by Nihon Kohden Corporation, "TB-612T"). The specimen was held in a Krebs solution which had been saturated with 95% oxygen and 5% carbon dioxide gas, and warmed to 37°C. Then, changes in isometric tension caused by adding various drugs were recorded on a pen-writing oscillograph (manufactured by Rikadenki Kogyo Co. Ltd, "R-64").

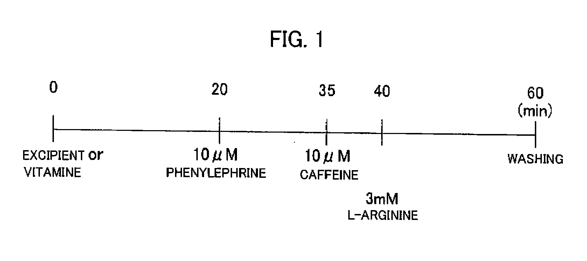

Fig. 1 illustrates an administration schedule of the various drugs. First, vitamin C was added at a concentration of 0.1 mM (Example 1), 1 mM (Example 2) and 10 mM (Example 3). Furthermore, the excipient alone was added in Comparative Example 1. Twenty minutes later, 10 µM phenylephrine was added thereto. Moreover, 10 µM caffeine was added 15 minutes later, and then 3 mM L-arginine was added five minutes afterwards. In addition, 20 minutes later, the drugs were rinsed away, and the degree of relaxation was determined. The test was carried out using five rabbits, and the evaluation was made with a mean value.

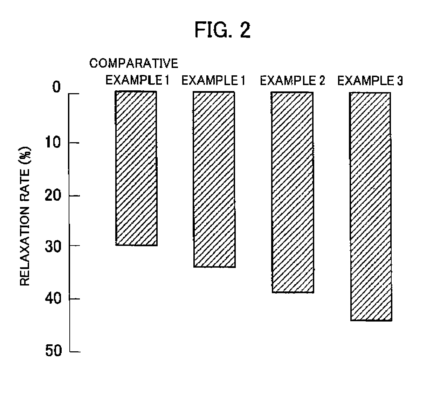

The results of Examples 1 to 3 and Comparative Example 1 are shown in Table 1, and Fig. 2. When 10 µM caffeine and 3 mM L-arginine were added under contraction induced by 10 µM phenylephrine, the percent relaxation rate was 30.6 +/-1.7% (the value to the left of "+/-" indicating the mean value of the five animals, while the value to the right thereof denoting the standard error, hereinafter the same applies) provided that the contraction induced by phenylephrine accounts for 100 as demonstrated in Comparative Example 1. Accordingly, the relaxation was evidently evoked. Furthermore, in Examples 1 to 3 in which vitamin C was added, the relaxation rate was increased with an increase of the vitamin C concentration. Hence, pretreatment with vitamin C significantly enhanced the relaxation induced by caffeine and L-arginine.

**[Table 1]**

| Ascorbic acid conc. (mM) | Comparative Example 1 | Example 1 | Example 2 | Example 3 |
|---|---|---|---|---|
| | 0 | 0.1 | 1 | 10 |
| 1 | 26.4 | 30.4 | 33.0 | 38.0 |
| 2 | 28.4 | 32.7 | 35.5 | 40.9 |
| 3 | 31.8 | 36.6 | 39.8 | 45.8 |
| 4 | 29.9 | 34.4 | 37.4 | 43.1 |
| 5 | 36.3 | 41.7 | 45.4 | 52.3 |
| Mean +/- SE | 30.6 +/- 1.7 | 35.2 +/- 1.9 | 38.2 +/- 2.1 | 44.0 +/- 2.4 |
| p value | - | NS | P<0.05 | P<0.005 |

### Examination of Effects of Added Vitamin E (VE, α-tocopherol) on Rabbit Penile Corpus cavernosum Dilator Response

### Examples 4 to 6

Examination was performed in a similar manner to Examples 1 to 3 except that vitamin C was changed to vitamin E, and the concentration of the added vitamin E was 1 µM (Example 4), 10 µM (Example 5), and 100 µM (Example 6).

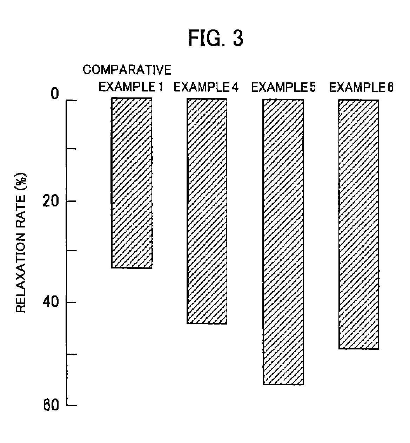

The results are shown in Table 2 and Fig. 3. The relaxation rate was increased in Examples 4 to 6, in which vitamin E was added, as compared with Comparative Example 1, whereby significant enhancement by vitamin E was ascertained.

**[Table 2]**

| α-tocopherol conc. (µM) | Comparative Example 1 | Example 4 | Example 5 | Example 6 |
|---|---|---|---|---|
| | 0 | 0 1 | 10 | 100 |
| 1 | 29.4 | 41.2 | 51.4 | 45.0 |
| 2 | 35.0 | 49.0 | 61.2 | 53.5 |
| 3 | 30.5 | 92.7 | 53.4 | 46.7 |
| 4 | 33.9 | 47.5 | 59.3 | 51.9 |
| 5 | 31.7 | 44.4 | 55.5 | 48.5 |
| mean +/- SE | 32.1 1.0 +/- | 45.0 1.5 +/- | 56.2 1.8 +/- | 49.1 1.6 +/- |
| p value | - | P<0.005 | P<0.005 | P<0.005 |

### Examination of Effects of Added Folic Acid (FA) on Rabbit Penile Corpus cavernosum Dilator Response

### Examples 7 to 9

Examination was performed in a similar manner to Examples 1 to 3 except that vitamin C was changed to folic acid, and the concentration of the added folic acid was 0.01 mM (Example 7), 0.1 mM (Example 8), and 1 mM (Example 9).

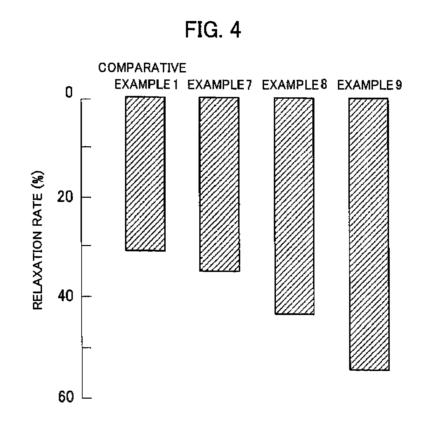

The results are shown in Table 3 and Fig. 4. The relaxation rate was increased in Examples 7 to 9 in which folic acid was added, as compared with Comparative Example 1, and thus a significant enhancement by folic acid was ascertained.

**[Table 3]**

| Folic acid conc. (mM) | Comparative Example 1 | Example 7 | Example 8 | Example 9 |
|---|---|---|---|---|
| | 0 | 0.01 | 0.1 | 1 |
| 1 | 28.0 | 33.0 | 41.4 | 53.2 |
| 2 | 34.4 | 40.6 | 50.9 | 65.4 |
| 3 | 31.8 | 37.5 | 47.1 | 60.4 |
| 4 | 26.2 | 30.9 | 38.8 | 49.8 |
| 5 | 29.0 | 34.2 | 42.9 | 55.1 |
| Mean +/- SE | 29.9 +/- 1.4 | 35.2 +/- 1.7 | 44.2 2.1 +/- | 56.8 2.8 +/- |
| p value | - | P<0.05 | P<0.005 | P<0.005 |

### Examination of Effects of Added Mixed Agent on Rabbit Penile Corpus cavernosum Dilator Response

### Examples 10 to 15

In Examples 10 to 15, concentrations of added vitamins were adjusted to 100 µM vitamin C, 0.1 µM vitamin E and 1 µM folic acid, and blended as shown in Table 4. They were compared with Comparative Example 1 as a control.

The results are shown in Table 4 and Fig. 5. In Examples 10 to 12, the concentration of each added vitamin was low as not to affect the relaxation caused by the addition of only caffeine and L-arginine as shown in Comparative Example 1. Meanwhile, in Examples 13, 14, and 15 in which any two of vitamin C, vitamin E, and folic acid were mixed, each concentration was the same as that in Examples 10 to 12. However, the relaxation rate was markedly increased: 48.9 +/-2.7% in Example 13, and 58.1 +/- 3.2% in Example 14. Furthermore, a significant increase was ascertained also in Example 15, although the extent was as low as 36.9% +/- 1.6%.

**[Table 4]**

| | Comparative Example 1 | Example 10 | Example 11 | Example 12 | Example 13 | Example 14 | Example 15 |
|---|---|---|---|---|---|---|---|
| | C+LA (10µM) +(3mM) | 100µM VC | 0.1µM VE | 1µM FA | VC+FA | VE+FA | VC+VE |
| | | C+LA | C+LA | C+LA | C+LA | C+LA | C+LA |
| 1 | 26.4 | 30.4 | 31.1 | 31.7 | 42.2 | 50.2 | 33.1 |
| 2 | 28.4 | 32.7 | 33.6 | 32.5 | 45.4 | 54.0 | 34.6 |
| 3 | 31.8 | 36.6 | 32.4 | 33.0 | 50.9 | 60.4 | 37.5 |
| 4 | 29.9 | 34.4 | 31.0 | 30.0 | 47.8 | 56.8 | 42.7 |
| 5 | 36.3 | 41.7 | 33.0 | 37.8 | 58.1 | 69.0 | 36.8 |
| Mean | 30.6 | 35.2 | 32.2 | 33.0 | 48.9 | 58.1 | 36.9 |
| +/- | +/- | +/- | +/- | +/- | +/- | +/- | +/- |
| SE | 1.7 | 1.9 | 0.5 | 1.3 | 2.7 | 3.2 | 1.6 |
| P value | - | NS | NS | NS | P< 0.005 | p< 0.005 | P<0.05 |

### Examination of Effects on Hypertension

### Example 16

To a rocking mixer (manufactured by Shang Yuh Machine Co., Ltd.) were added 0.004 kg of vitamin E (70%) (manufactured by Kasano Kosan Corporation), 0.011 kg of folic acid (DSM Nutrition Japan K.K.), 1.11 kg of anhydrous caffeine (manufactured by Shizuoka Coffein Co., Ltd.), 33.33 kg of L-arginine (manufactured by Kyowa Hakko Kogyo Co., Ltd.), 1.039 kg of crystalline cellulose (Vivapur 101; manufactured by Rettenmeier GmbH), and 0.5 kg of sucrose fatty acid ester (S-307 F; manufactured by Mitsubishi-Kagaku Foods Corporation), which were then mixed for 10 min. After the mixing, classification and the presence of lump were visually confirmed. Each 360 mg of the thus resulting mixture was filled in a capsule to produce an encapsulated formulation.

Ten panelists continued to take two to four capsules of Example 16 after meals, three to eight capsules per day, for about two months. Then, blood pressure before and after the administration was compared to make an evaluation. The results are shown in Table 5.

**[Table 5]**

| | Age /Sex | Dose per day | Before administration | | After 1 month | | After 2 months | | Remarks |
|---|---|---|---|---|---|---|---|---|---|
| | | | Systolic BP | Diastolic BP | Systolic BP | Diastolic BP | Systolic BP | Diastolic BP | |
| (1) | 56 /Male | 8 capsules {4 capsules * twice} | 132 | 79 | 123-132 | 75-85 | 134-139 | 87-90 | During dosing Period (two months), BP was steady. |
| (2) | 63 /Male | 6 capsules (2 capsules * three times) | 140 | 90 | 139 | 79 | 140 | 79 | |
| (3) | 43 /Male | 8 capsules (4 capsules * twice) | >140 | 95-110 | 120-136 | 83-89 | - | - | |
| (4) | 58 /Male | 8 capsules (4 capsules * twice) | 140-150 | - | 120-126 | 82-86 | - | - | |
| (5) | 63 /Male | (8 capsules * once) (B capsules * once) | 140-160 | 70-110 | 140-160 | 70-110 | - | - | |
| (6) | 65 /Male | (3 capsules * once) (3 capsules * once) | 150-160 | 105-108 | 140-145 | 90-95 | 135-190 | 80-90 | |
| (7) | 66 /Male | 4 capsules (4 capsules * once) | 153-155 | 90 | - | - | 145-148 | 71-73 | |
| (8) | 57 /Male | 6 capsules (3 capsules * twice) | 158-183 | 90-102 | 147-165 | 85-91 | 142-198 | 81-87 | |
| (9) | 57 /Male | 6 capsules (2 capsules * three times) | 110-120 | 70-80 | 110-120 | 70-80 | - | - | |
| (10) | 61 /Male | 4 to 9 capsules | 130-150 | 80-100 | 130-150 | 80-100 | - | - | |

As seen from Table 5, blood pressures of the male specimens (2), (3), (4), (6), (7), and (8) were lowered by taking the capsule, and thus the effect on hypertension could be verified. In particular, the male specimen (8) with high blood pressure showed a noteworthy effect. Although the male specimen (1) showed no change in the blood pressure, his blood pressure was stabilized while the period during which the capsule had been taken. In addition, even though the male specimen (9) with blood pressure in a normal range took the capsule, the effect was not shown. However, it was also ascertained that side effects were not caused, revealing absence of the problem. From the foregoing, it was verified that the capsule in which the beverage composition including L-arginine of the present invention was used was effective in treating hypertension.

## Claims

1. A pharmaceutical composition comprising the following components:
(A) one of L-arginine and a salt thereof;
(B) one of caffeine and a salt thereof; and
(C) at least one substance selected from the group consisting of one of vitamin C and a derivative thereof, one of vitamin E and a derivative thereof, and one of folic acid and a derivative thereof.

2. A pharmaceutical composition according to claim 1, wherein at least two substances are used in combination as the component (C).

3. A pharmaceutical composition according to claim 2, wherein at least the one of vitamin E and a derivative thereof, and the one of folic acid and a derivative thereof are used in combination as the component (C).

4. A pharmaceutical composition according to any one of claims 1 to 3 used for at least one of the prevention, amelioration and treatment of at least one disease selected from the group consisting of arteriosclerosis, angiectasia, hyperlipemia, climacteric disorder, diabetes, angina pectoris, hypertension, erectile dysfunction, thrombosis, immunodeficiency, dementia, gestosis, respiratory failure and hemorrhoid.

5. A pharmaceutical composition according to any one of claims 1 to 4, comprising: 0.05 to 67 moles of the component (B); and at least one selected from the group consisting of one of 0.05 to 67 moles of vitamin C and a derivative thereof, one of 0.0005 to 67 moles of vitamin E and a derivative thereof, and one of 0.005 to 67 moles of folic acid and a derivative thereof as the component (C), per 100 moles of the component (A).

6. A beverage composition comprising the following components:
(A) one of L-arginine and a salt thereof;
(B) one of caffeine and a salt thereof; and
(C) at least one substance selected from the group consisting of one of vitamin C and a derivative thereof, one of vitamin E and a derivative thereof, and one of folic acid and a derivative thereof.

7. A beverage composition according to claim 6, wherein at least two substances are used in combination as the component (C).

8. A beverage composition according to claim 7, wherein at least the one of vitamin E and a derivative thereof, and the one of folic acid and a derivative thereof are used in combination as the component (C).

9. A beverage composition according to any one of claims 6 to 8, comprising at least one selected from the group consisting of 0.05 to 67 moles of the component (B); and 0.05 to 67 moles of one of vitamin C and a derivative thereof, 0.0005 to 67 moles of one of vitamin E and a derivative thereof, and 0.005 to 67 moles of one of folic acid and a derivative thereof as the component (C), per 100 moles of the component (A).
